**Europäisches Patentamt**

**European Patent Office**

**Office européen des brevets**

(11) Veröffentlichungsnummer: **0 198 800**
**B1**

(12) **EUROPÄISCHE PATENTSCHRIFT**

(45) Veröffentlichungstag der Patentschrift:
**24.08.88**

(21) Anmeldenummer: **86810171.8**

(22) Anmeldetag: **11.04.86**

(51) Int. Cl.⁴: **C 07 C 143/48**

(54) Verfahren zur Herstellung von **1-Diazo-2-naphthol-4-sulfonsäure.**

(30) Priorität: **17.04.85 CH 1636/85**

(43) Veröffentlichungstag der Anmeldung:
**22.10.86 Patentblatt 86/43**

(45) Bekanntmachung des Hinweises auf die Patenterteilung:
**24.08.88 Patentblatt 88/34**

(84) Benannte Vertragsstaaten:
**CH DE FR GB IT LI**

(56) Entgegenhaltungen:
**DE - C - 171 024**
**DE - C - 175 593**
**DE - C - 178 621**
**DE - C - 189 179**

(73) Patentinhaber: **CIBA-GEIGY AG, Klybeckstrasse 141,**
**CH-4002 Basel (CH)**

(72) Erfinder: **Förtsch, Bruno, Dr., Eggweg,**
**CH-4433 Ramlinsburg (CH)**

## Beschreibung

Die Erfindung betrifft ein Verfahren zur Herstellung von 1-Diazo-2-naphthol-4-sulfonsäure durch Diazotieren von 1-Amino-2-naphthol-4-sulfonsäure in schwach saurem Medium in Gegenwart katalytischer Mengen eines Eisensalzes.

1-Diazo-2-naphthol-4-sulfonsäure ist ein wichtiges Farbstoffzwischenprodukt und dient als Diazokomponente zur Herstellung einer ganzen Reihe von Textil- und Lederfarbstoffen. Die um die Jahrhundertwende von Böniger durchgeführten Versuche zur Diazotierung von 1-Amino-2-naphthol-4-sulfonsäure lieferten zunächst nicht die gewünschte Diazoverbindung, sondern führten zur 1,2-Naphthochinon-4-sulfonsäure [Chem. Ber. 27, 3050 (1894)]. Erst die Beobachtung, dass sich die Oxidationsreaktion durch Zusatz von Kupfer- oder Eisensalzen zugunsten der Diazotierung zurückdrängen lässt (DE-B-171 024), führte zu einer breiten Anwendung der 1-Amino-2-naphthol-4-sulfonsäure als Diazokomponente in der Farbstoffchemie. Die genaue Wirkungsweise der Metallsalze bei der Diazotierung ist auch heute noch nicht bekannt. Man nimmt jedoch an, dass die Kupfer- oder Eisenionen koordinativ über die Hydroxy- und Aminogruppe an die 1-Amino-2-naphthol-4-sulfonsäure gebunden werden und so die Hydroxygruppe vor einem oxidativen Angriff durch die salpetrige Säure schützen.

Da heute die Abwasserbestimmungen zunehmend strenger werden, ist man bemüht, die Belastung des Abwassers durch Schwermetallsalze möglichst gering zu halten. Im Zusammenhang mit der hier beschriebenen Reaktion war bereits bekannt, dass der gewünschte Effekt, die Verhinderung der Oxidation der Aminonaphtholsulfonsäure, bereits mit weniger, als der äquimolaren Menge an Metallsalz erreicht wird. Gefunden wurde nun, dass sich die erforderliche Metallsalzmenge noch weiter reduzieren lässt, wobei im Falle der Verwendung von Eisensalzen überraschenderweise ein optimaler Konzentrationsbereich durchlaufen wird, der weit unter der stöchiometrisch erforderlichen Menge liegt.

Gegenstand der vorliegenden Erfindung ist somit ein Verfahren zur Herstellung von 1-Diazo-2-naphthol-4-sulfonsäure durch Diazotieren von 1-Amino-2-naphthol-4-sulfonsäure in schwach saurem Medium, in Gegenwart von Eisenionen, das dadurch gekennzeichnet ist, dass man bezogen auf 1 Mol 1-Amino-2-naphthol-4-sulfonsäure 1 bis 30 mMol einer Eisenverbindung einsetzt.

Die 1-Amino-2-naphthol-4-sulfonsäure, die z.B. ausgehend von β-Naphthol über das 1-Nitroso-2-naphthol und die 1-Hydroxylimino-2-tetralon-4-sulfonsäure in guter Ausbeute leicht zugänglich ist [siehe Ullmanns Enzyklopädie der technischen Chemie, 4. Auflage, Band 17 (1979) Seite 100], kann sowohl als freie Säure, als auch in Form des Alkalimetallsalzes, z.B. des Natrium- oder Kaliumsalzes eingesetzt werden. Das Ausgangsmaterial kann in getrockneter Form oder auch in Form eines mehr oder weniger wasserhaltigen Presskuchens verwendet werden.

Die Diazotierung wird in einem schwach sauren wässrigen Medium durchgeführt, wobei unter dem Begriff schwach sauer ein pH-Wert im Bereich von 3,5 bis 6, insbesondere ein pH-Wert von 4 bis 5 zu verstehen ist. Eingestellt wird der pH-Wert zweckmässigerweise mittels verdünnter Schwefelsäure.

Anstelle einer Mineralsäure kann zum Ansäuern des Reaktionsmediums aber z.B. auch Essigsäure oder Oxalsäure verwendet werden. Um bei einem möglichst konstanten pH-Wert zu arbeiten, empfiehlt sich der Zusatz eines puffernd wirkenden Metallsalzes, z.B. eines Alkalimetallsalzes einer schwachen organischen Säure. Insbesondere arbeitet man in einer acetatgepufferten schwefelsauren Lösung, die einen pH-Wert von 4 bis 4,5 aufweist.

Als Diazotierungsmittel verwendet man in erster Linie Alkalinitrite, z.B. Natriumnitrit, das zweckmässigerweise in Form einer wässrigen Lösung langsam kontinuierlich oder diskontinuierlich der im Reaktionsmedium suspendierten 1-Amino-2-naphthol-4-sulfonsäure zugesetzt wird. Ferner kommen als Diazotierungsmittel die Salpetrigsäureester primärer und sekundärer aliphatischer Alkohole, wie z.B. Methylnitrit, Äthylnitrit, Amylnitrit, Butylnitrit oder Isopentylnitrit in Frage. Das Diazotierungsmittel wird in einem geringen Überschuss bezogen auf die stöchiometrisch erforderliche Menge eingesetzt.

Erfindungswesentlich ist, dass die Diazotierung in Gegenwart von 1 bis 30 mMol einer Eisenverbindung pro Mol Aminonaphtholsulfonsäure durchgeführt wird. Als Eisenverbindungen kommen beispielsweise Eisensalze, Eisenhydroxide, Eisenoxide oder auch Eisenkomplexe in Frage. Als Salze z.B. Sulfate, Nitrate, Chloride Bromide, Jodide oder Eisencarbonate; als Oxide bzw. Hydroxide z.B. basische Eisenoxidhydrate. Bevorzugt verwendet werden Eisen-II-salze, insbesondere das Eisen-II-sulfat, z.B. wasserfreies Eisen-II-sulfat oder auch kristallwasserhaltiges Eisen-II-sulfat, wie z.B. das Heptahydrat. Die Eisenverbindungen können sowohl in fester Form, als auch in gelöster Form, z.B. als wässrige oder schwefelsaure Lösung verwendet werden.

Bezogen auf ein Mol zu diazotierende 1-Amino-2-naphthol-4-sulfonsäure verwendet man bevorzugt 6 bis 25 mMol an Eisenverbindung. Bei Einhalten dieses Konzentrationsbereichs erhält man die 1-Diazo-2-naphthol-4-sulfonsäure in einer Ausbeute von 95 bis fast 100%. Bei Verwendung von Eisen-II-sulfat erzielt man eine optimale Ausbeute (96 bis 99% der Theorie), wenn man auf 1 Mol Diazonaphtholsulfonsäure 15 bis 20 mMol Eisen-II-sulfat einsetzt. Bei Verwendung von weniger als 1 mMol bzw. mehr als 30 mMol Eisenverbindung pro Mol Aminonaphtholsulfonsäure fällt die Ausbeute an Diazonaphtholsulfonsäure deutlich ab. Man erhält dann die gewünschte Diazoverbindung nur noch in Ausbeuten von weniger als 90%.

Durchgeführt wird die Diazotierung bei einer Temperatur, die zweckmässigerweise im Bereich von 0° bis 60°C liegt; vorteilhaft arbeitet man bei einer Temperatur von 10 bis 30°C. Die Diazotierung kann auch bei tieferen Temperaturen durchgeführt werden, was jedoch zu unwirtschaftlich langen Reaktionszeiten führt.

Die Reaktionszeit beträgt bei einem 100molaren Ansatz ca. 20 Minuten bis 1 Stunde. Der Fortgang der Reaktion lässt sich leicht anhand der Abnahme

3        0 198 800        4

der im Reaktionsmedium suspendierten 1-Aminonaphtholsulfonsäure verfolgen. Man erhält schliesslich eine wässrige Reaktionslösung, die gegebenenfalls noch einen geringen Anteil an unlöslichen Nebenprodukten enthält. Diese werden zweckmässigerweise mittels Klärfiltration entfernt.

Durch Versetzen der klaren Reaktionslösung mit Schwefelsäure wird die Diazonaphtholsulfonsäure in kristalliner Form ausgefällt. Um eine gut filtrierbare Kristallform zu erhalten, erweist es sich als zweckmässig, die zur vollständigen Fällung notwendige Säuremenge langsam über einen Zeitraum von mehreren Stunden zuzusetzen und dabei die Reaktionsmasse ständig zu rühren. Isoliert wird die ausgefällte Diazonaphtholsulfonsäure nach bekannten Methoden z.B. durch Filtration, Zentrifugieren oder Dekantieren. Als vorteilhaft erweist sich eine Kombination aus Filtration und Zentrifugieren. Das Produkt kann in Form des feuchten Filterkuchens direkt mit geeigneten Kupplungskomponenten zu Azofarbstoffen verarbeitet werden, insbesondere zu metallisierbaren Azofarbstoffen für die Wollfärberei.

Das folgende Beispiel dient der Veranschaulichung der Erfindung; Teile bedeuten Gewichtsteile und Prozente Gewichtsprozente.

*Beispiel*

In einem Rührkessel werden 100 Teile Wasser vorgelegt und darin 30 Teile 1-Amino-2-naphthol-4-sulfonsäure und 7,24 Teile Natriumacetat suspendiert. Dann gibt man noch 25 Teile Wasser zu und stellt den pH-Wert der Suspension durch Zusatz von 33%iger Schwefelsäure, ca. 5 Teile, auf 4,2 ein. Anschliessend werden 0,62 Teile Eisen-II-sulfat-Heptahydrat (FeSO$_4 \cdot$7H$_2$O) gelöst in 2,3 Teilen Wasser zugegeben; das entspricht einer Menge von 17,22 mMol Eisensulfat pro Mol 1-Amino-2-naphthol-4-sulfonsäure. Schliesslich gibt man noch 0,073 Teile eines Antischaummittels auf Basis von 2-Äthyl-n-hexanol hinzu, kühlt den Reaktorinhalt auf 10°C ab und lässt dann unter guter Durchmischung 37,5 Teile einer 4n Natriumnitritlösung zulaufen. Als Antischaummittel kann beispielsweise auch ein Acetylen-Glykol, wie 3,6-Dimethyl-4-octin-3,6-diol verwendet werden. Durch Kühlung wird die Temperatur im Bereich von 10 bis 30°C gehalten. Zur Vervollständigung der Diazotierungsreaktion rührt man die Reaktionslösung nach Zugabe des Natriumnitrits noch eine Stunde, gibt dann ein Filterhilfsmittel hinzu und befreit die Reaktionslösung mittels Klärfiltration von unlöslichen Nebenprodukten. Dann versetzt man die Reaktionslösung unter Rühren mit 71,2 Teilen 33%iger Schwefelsäure und fällt so die 1-Diazo-2-naphthol-4-sulfonsäure in kristalliner Form aus. Das Produkt wird abfiltriert und ein Teil des Restwassers durch Zentrifugieren entfernt. Man erhält 41,2 Teile wasserfeuchtes Rohprodukt mit einem Gehalt an 1-Diazo-2-naphthol-4-sulfonsäure von 73,2%, das entspricht einer Ausbeute von 96,1%.

Die Analyse des Produkts erfolgt mit Hilfe der Flüssigkeits-Chromatographie; das Produkt zeigt hier die gleiche Retentionszeit, wie eine authentische Probe der 1-Diazo-2-naphthol-4-sulfonsäure.

Die Menge an nichtumgesetzter 1-Amino-2-naphthol-4-sulfonsäure im Rohprodukt liegt unter 0,1%.

Die 1-Diazo-2-naphthol-4-sulfonsäure kann ohne weitere Reinigung direkt zur Herstellung von Azofarbstoffen verwendet werden. Vergleichbare Ergebnisse erhält man, wenn man anstelle des Eisen-II-sulfats eine äquimolare Menge an Eisen-II-chlorid oder Eisenoxidhydrat verwendet. Die gleiche oder eine nur etwas geringere Ausbeute erzielt man wenn man anstelle von 17,22 mMol Eisen-II-salz 12 oder 20 mMol an Eisen-II-salz einsetzt, bezogen auf 1 Mol 1-Amino-2-naphthol-4-sulfonsäure.

**Patentansprüche**

1. Verfahren zur Herstellung von 1-Diazo-2-naphthol-4-sulfonsäure durch Diazotieren von 1-Amino-2-naphthol-4-sulfonsäure in schwach saurem Medium, in Gegenwart von Eisenionen, dadurch gekennzeichnet, dass man bezogen auf 1 Mol 1-Amino-2-naphthol-4-sulfonsäure 1 bis 30 mMol einer Eisenverbindung einsetzt.

2. Verfahren gemäss Anspruch 1, dadurch gekennzeichnet, dass man als Eisenverbindung ein Eisen-II-salz verwendet.

3. Verfahren gemäss Anspruch 2, dadurch gekennzeichnet, dass man Eisen-II-sulfat verwendet.

4. Verfahren gemäss den Ansprüchen 1 bis 3, dadurch gekennzeichnet, dass man bezogen auf 1 Mol 1-Amino-2-naphthol-4-sulfonsäure 6 bis 25 mMol an Eisenverbindung einsetzt.

5. Verfahren gemäss Anspruch 3, dadurch gekennzeichnet, dass man pro Mol 1-Amino-2-naphthol-4-sulfonsäure 15 bis 20 mMol an Eisen-II-sulfat verwendet.

6. Verwendung der gemäss Anspruch 1 erhaltenen 1-Diazo-2-naphthol-4-sulfonsäure zur Herstellung von Textil- und Lederfarbstoffen.

**Claims**

1. A process for the preparation of 1-diazo-2-naphthol-4-sulfonic acid by diazotising 1-amino-2-naphthol-4-sulfonic acid in weakly acid medium and in the presence of iron ions, which process comprises the use of 1 to 30 millimoles of an iron compound, based on 1 mole of 1-amino-2-naphthol-4-sulfonic acid.

2. A process according to claim 1, which comprises the use of an iron(II) salt as the iron compound.

3. A process according to claim 2, which comprises the use of iron(II) sulfate.

4. A process according to any one of claims 1 to 3, wherein 6 to 25 millimoles of iron compound are used per mole of 1-amino-2-naphthol-4-sulfonic acid.

5. A process according to claim 3, wherein 15 to 20 millimoles of iron(II) sulfate are used per mole of 1-amino-2-naphthol-4-sulfonic acid.

6. The use of the 1-diazo-2-naphthol-4-sulfonic acid obtained according to claim 1 for the preparation of textile and leather dyes.

3

## Revendications

1. Procédé pour la préparation de l'acide 1-diazo-2-naphtol-4-sulfonique par diazotation de l'acide 1-amino-2-naphtol-4-sulfonique en milieu légèrement acide, en présence d'ions fer, caractérisé par le fait que l'on utilise de 1 à 30 mmoles d'un composé à base de fer, par rapport à 1 mole d'acide 1-amino-2-naphtol-4-sulfonique.

2. Procédé selon la revendication 1, caractérisé par le fait que l'on utilise un sel ferreux en tant que composé à base de fer.

3. Procédé selon la revendication 2, caractérisé par le fait que l'on utilise du sulfate ferreux.

4. Procédé selon les revendications 1 à 3, caractérisé par le fait que l'on utilise de 6 à 25 mmoles de composé à base de fer, par rapport à 1 mole d'acide 1-amino-2-naphtol-4-sulfonique.

5. Procédé selon la revendication 3, caractérisé par le fait que l'on utilise de 15 à 20 mmoles de sulfate ferreux par mole d'acide 1-amino-2-naphtol-4-sulfonique.

6. Utilisation de l'acide 1-diazo-2-naphtol-4-sulfonique obtenu selon la revendication 1, pour la préparation de colorants pour textiles et pour cuir.